# EUROPEAN PATENT APPLICATION

(11) **EP 4 344 772 A2**
(43) Date of publication of application: **03.04.2024**
(21) Application number: 24156972.2
(22) Date of filing: 12.02.2024
(51) Int. Cl.: B01J 20/26, B01J 20/28, C12M 1/00, C12N 11/089

(54) **LACTATE-ADSORBING SORBENT MATERIAL**

(30) Priority: 10.03.2023 US 202363489637 P
(71) Applicant: Sartorius Stedim Cellca GmbH, 89081 Ulm (DE); Purdue Research Foundation, Indiana 47906 (US)
(72) Inventor: AKBARI, Samin, Winchester, 2210 (US); ZEHE, Christoph, 89584 Ehingen (DE); WEI, Alexander, West Lafayette, 47906 (US)
(74) Representative: Müller-Boré & Partner Patentanwälte PartG mbB

(57) **Abstract**

A lactate-adsorbing sorbent material for application in a bioreactor is provided. The sorbent material comprises an active material having a polymeric backbone comprising one or more kinds of functional groups selected from boronic acid groups and boronic acid ester groups. The sorbent material can be in the form of a semi-permeable pouch containing the active material, in the form of a reactor wall coating or in the form of a tablet.

## Description

The present invention relates to a lactate-adsorbing sorbent material for application in a bioreactor, a method of cultivating cells in a bioreactor and a method of using a sorbent material in a bioreactor.

A current trend in bioreactor design is to employ single-use bioprocessing bags that are mechanically sealed to avoid contamination. Ideally, these sterile bags should be minimally disturbed for the duration of the bioprocess (up to several weeks), however cell cultures generate byproducts, such as lactate, whose buildup can deteriorate their health and productivity. Removing waste products and resupplying important nutrients is typically accomplished by replacing cell culture media, which is expensive and also time-consum ing.

Media exchange is performed at least once for cell cultures with incubation times on the order of weeks. Perfusion bioreactors have been designed that enable partial exchange of culture media without compromising sterility, however the technological burden and cost of the bioprocess workflow increases significantly. Complex bioreactor technology requires extra training and increases the consumption of expensive cell culture media, which hinders their adaptation in operations requiring good manufacturing practices (GMP).

One such byproduct, which is continuously formed in a cell culture, is lactate. Lactate becomes increasingly detrimental to cell health with increasing concentrations. There are sorbents specifically engineered for lactate adsorption. However, no commercial products that use chemical sorption as a mechanism to remove waste metabolites in *situ* from cell culture media are available.

Lactate ions have, for example, been removed by passing fermentation broth through a column of activated charcoal mixed with polyvinylpyridine, whose cost and mode of operation are impractical for single-use systems (Chen, C.; Ju, L. K., Coupled lactic acid fermentation and adsorption. Appl. Microbiol. Biotechnol. 2002, 59, 170-174). Certain forms of biochar prepared by hydro-thermal carbonization have been found to absorb lactate from fermentation broth up to 140 mg/g, although no selectivity or cytotoxicity tests were described (Laube, H.; Reza, M. T., Application of biosorbents for ion removal from sodium lactate fermentation broth. J. Environ. Chem. Eng. 2016, 4, 10-19). Most recently, certain layered inorganic materials have shown some promise for lactate adsorption from cell culture media, although saturation limits were not clearly reported (Kameda, T.; Kikuchi, H.; Kitagawa, F.; Kumagai, S.; Saito, Y.; Kondo, M.; Jimbo, Y.; Yoshioka, T., Lactate adsorption by layered double hydroxides in aqueous solution and cell culture medium. Colloid. Surf. A 2021, 612, 125975).

In view of the above, it is an object of the present invention to provide a labor-free alternative for maintaining healthy cell cultures under sterile conditions.

As a solution to the described challenges the inventors of the present invention propose that a lactate-adsorbing sorbent material could solve some of the above discussed issues.

In particular, the present invention provides a lactate-adsorbing sorbent material for application in a bioreactor comprising an active material, the active material having a polymeric backbone comprising one or more kinds of accessible functional groups selected from boronic acid groups and boronic acid ester groups, wherein the sorbent material is in the form of a semi-permeable pouch, bag or sachet containing the active material, in the form of a reactor wall coating, in the form of a patch fixed to the reactor wall, in the form of a tablet, in the form of porous or non-porous beads or in the form of a membrane or filter cartridge floating on or immersed in the cell culture.

The active material of the lactate-adsorbing sorbent material according to the present invention comprises one or more kinds of accessible functional groups selected from boronic acid groups and boronic acid ester groups. In a case where the active material carries boronic acid ester groups, the ester bond may be hydrolytically or enzymatically cleaved during application to provide free boronic acid groups. According to the present invention, an accessible functional group is available for a chemical reaction and does not form part of the polymeric backbone of the active material.

Boronic acid groups are capable of binding molecules having cis-diols or α-hydroxy acids, which is the case for lactic acid. Specifically, boronic acid groups are capable of binding two lactate molecules per boronic acid group, one as part of the normal electron sextet and the second one as part of the electron octet extension at the boron atom. Such a binding regime is depicted in the following scheme:

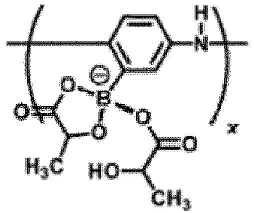

Providing a compound with boronic acid groups to a cell culture enables the adsorption of lactate ions. Lactate is continuously formed in cell cultures as a metabolite, which has an increasingly disruptive effect on the health of the cell culture. By adsorbing lactate and thereby lowering its concentration in the cell culture medium, the cytotoxic properties of excess lactate in the medium can be avoided. This allows for an increased running time of cell cultures, increased viability of the cells (by inhibiting cell death), increased cell count and higher yields of target proteins produced by the cell cultures in batch or semi-batch reactors.

In a preferred embodiment, the lactate-adsorbing sorbent material is a lactate-specific sorbent material. This means that in an environment, where lactate and other cis-diols or α-hydroxy acids are present at the same concentration, a higher proportion of lactate is adsorbed to the sorbent material compared to the other cis-diols or α-hydroxy acids.

The lactate-adsorbing material according to the present invention has a polymeric backbone. By having the functional groups attached to a polymer backbone, the active material can be separated more easily from the cell culture after the cultivation time has expired, compared to a case in which low molecular weight (LMW) boronic acid molecules or LMW boronic acid ester molecules are provided to the cell culture. When the sorbent material is present in the form of a semi-permeable pouch, bag, sachet, tablet, bead, membrane or filter cartridge, the active material can be easily removed from the cell culture after it has reached its sorption capacity. For example, porous or non-porous beads can be removed from the cell culture by simple filtration. In the case of a reactor wall coating or a patch fixed to the reactor wall, these remain with the reactor when the culture broth is removed from the reactor.

Furthermore, the active material can consist of individual polymeric backbone strands or several polymeric backbone strands can be bound to a common carrier, such as porous or non-porous beads.

With regard to the use of porous or non-porous beads as carrier for the active material, these can be beads with sizes or porosities being in the micrometer range (microbeads) or nanometer range (nanobeads). The material of the beads is not particularly limited, as long as several strands of the polymeric backbone of the active material can be bound thereto. For example, the beads may be beads formed from metals, metal oxides or polymers. As polymers, polyolefins, aromatic polymers, polyacetals, polyamides, polyesters, polyurethanes, polycarbonates, polysiloxanes, and naturally occurring biopolymers such as polysaccharides, polypeptides, polyhydroxyalkanoates, polymerized lipids, and humic acids may be named. With regard to beads, a number of polymers can be prepared in latex form by emulsion polymerization.

In the case of porous beads, the beads are designed such that the pores are smaller in size than the beads, preferably by at least one order of magnitude. The lactate-adsorbing sorbent material can be part of the beads as part of a coating of the inner and/or outer surfaces of the beads with the coating being designed to be released or dissolved over the duration of the cell-culture process. In an alternative embodiment the lactate-adsorbing sorbent material is part of the core of the beads and protected by an outer coating in the form of a lid surrounding the core with the lid dissolving over time to release the lactate-adsorbing sorbent. The beads or parts thereof can be biodegradable, i.e. can be decomposed or broken up into their monomers or other products of decomposition by the impact of physiological conditions. For example, in the case of beads having a core-shell structure, where the active material resides in the core of the bead, the shell may be made of a biodegradable polymer, which breaks down in the cell culture and thereby makes the active material accessible to the cell culture only after a certain time.

Biodegradable polymers that can be used to form porous or non-porous beads with the intended size and/or porosity limitations are known in the art. For example, polyesters, polyethers and polyamides may be named as suitable classes of materials. These may be naturally occurring or be prepared synthetically. As naturally occurring biodegradable polymers polysaccharides and proteins may be named. Common synthetically prepared polymers are, for example, polyhydroxybutyrate and polylactic acid.

The kind of polymeric backbone used in the present invention is not particularly limited, as long as it is able to carry the functional groups. Furthermore, the polymeric backbone can be obtained by polymerizing monomer units already carrying the functional groups, or an already existing polymeric backbone can be grafted with the functional groups.

Suitable polymeric backbones include, but are not limited to, polyolefins, aromatic polymers, polyacetals, polyamides, polyesters, polyurethanes, polycarbonates, polysiloxanes, and naturally occurring biopolymers such as polysaccharides, polypeptides, polyhydroxyalkanoates, polymerized lipids, humic acids, and modified latex particles. For example, polymers derived from (meth)acrylic acid derivatives, styrene derivatives, substituted anilines and heterocycles such as pyrroles and thiophenes, or any monomers with pendant groups capable of adsorbing lactate ions may be used.

Polymers can be comprised of 100's to 10,000's of monomers. According to a preferred embodiment, each monomer has a lactate-adsorbing unit (highest sorption per mass), leading to a maximized sorption capacity.

According to the present invention, the polymeric material needs to be sufficiently interactive with water for lactate sorption or exchange to occur. In this regard, size (molecular weight) is less important although it has some effect on solvation, binding kinetics, and ease of handling.

Moreover, according to the present invention, the molecular architecture of the supporting polymer should be such that it does not produce leachates that might contaminate the media. The specific details depend on the mode of sorbent presentation. In one instance, if presented in a pouch with a semi-permeable membrane having a specific molecular weight cutoff (MWCO), the size of the supporting polymer should exceed the MWCO value. According to a preferred embodiment, MWCO values of semi-permeable membranes can range from 2 kDa to 100 kDa. In another instance, if presented as a microporous bead, the supporting polymer should have a minimum density of covalent crosslinks with other polymers to prevent its gradual desorption into the media.

According to a preferred embodiment of the present invention, the supporting polymer has sufficient resistance to fragmentation when exposed to sterilizing methods such as ionizing, x-ray, or ultraviolet radiation or autoclaving. In some cases, ionizing radiation can be used to crosslink such polymers and thereby increase its mechanical strength and molecular weight (MW) distribution. According to a preferred embodiment, there can be used polymer blends in which the primary (lactate-selective) polymer is strengthened by crosslinking with the secondary material, which can be performed as a distinct step in the manufacturing process or in situ during ionizing radiation.

According to the present invention, the distribution of lactate-selective units on the supporting polymer or polymer blend should be as high as possible, while maintaining sufficient chemical and physical integrity and compatibility with the media. For instance, polymers with aromatic rings can be designed so that each ring contains at least one boronic acid or ester unit.

According to a preferred embodiment of the present invention, the density of lactate-selective units on polymer backbones may vary between 0.1 and 10 wt%, preferably between 2 and 10 wt%, more preferably between 5 and 10 wt%, and even more preferably between 8 and 10 wt%.

The polymeric backbone can be a homopolymer consisting of one kind of monomer units or a copolymer combining two or more different monomer units. Accordingly, functional groups may be provided on every repeating unit of the polymer backbone or only on some of the repeating units. Such functionalized polymers can be prepared from their corresponding monomers containing lactate-selective functionality using known polymerization processes, or can be prepared by installing lactate-selective functionality post-polymerization.

In a preferred embodiment, at least 75% of the repeating units carry a lactate-binding functional group, more preferably at least 85%, even more preferably at least 95%. In a specific embodiment, 100% of the repeating units carry a functional group.

According to a specific embodiment, the polymeric backbone is an aniline-based polymer. For example, an active material according to the present invention can be obtained by polymerizing aminophenylboronate (APB) lactate ester to obtain polyaminophenyl boronic acid (PAPB), wherein the lactate ester group is a protective group for the subsequent polymerization reaction. In a specific embodiment, 3-aminophenylboronic acid is polymerized in the presence of sodium lactate to obtain PAPB according to chemical formula (1) below: 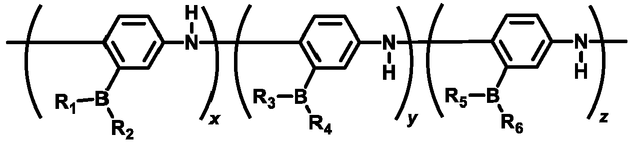 where residues attached to the same boron atom (e.g. R₁ and R₂) are either hydroxy groups or taken together form a boronate ester group. The indices x, y, and z represent the fraction of the respective monomer units relative to the whole polymer, where x + y + z equals 1.

The sorbent material is in the form of a semi-permeable pouch, bag or sachet containing the active material, in the form of a reactor wall coating, in the form of a patch fixed to the reactor wall, or in the form of a tablet. Alternatively, the sorbent material can be in the form of porous or non-porous beads or in the form of a membrane or filter cartridge floating on or immersed in the cell culture. Combinations of two or more of the aforementioned embodiments can be used in the cell culture.

By providing the sorbent material in one of the above forms to the cell culture, the lactate in the medium can diffuse towards or into the active material and react with boronic acids or esters to form a covalently bound lactate ester, thereby reducing the amount of free lactate in the medium.

Moreover, the sorbent material can be separated easily from the cell culture after the culture time has expired. In particular, in the case of a semi-permeable pouch, tablet, membrane or filter cartridge, these articles can be physically removed from the cell culture. Furthermore, in the case of a reactor wall coating or patch fixed to the reactor wall, the sorbent material remains on the reactor walls when the cell culture is removed. Porous or non-porous beads can be mechanically filtered off the cell culture to remove the active material.

According to the present invention, a tablet of the sorbent material comprises the active material compacted in a tablet shape, optionally with addition of a binder material. Such a compacted tablet does not dissolve or break up in the cell culture but remains as a unit. However, even in this compacted form, the functional groups of the active material remain accessible for small molecules, such as lactate.

A further advantage of providing the sorbent material in the form of a semi-permeable pouch, bag or sachet containing the active material is that a direct contact between the cells and the sorbent material can be avoided. Specifically, the semi-permeable pouch, bag or sachet is formed from a semi-permeable membrane that is permeable for small molecules but cannot be permeated by cells. In this way, the lactate from the cell culture can diffuse through the semi-permeable membrane and react with the functional groups on the active material inside the pouch.

According to a preferred embodiment, dialysis membranes with specific molecular cut off range can be used as semi-permeable membranes.

In particular, according to the present invention, for example dialysis bags with molecular weight cut off values MWCO ranging between 2 kDa and 100 kDa and/or membranes with MWCO ranging between 2 kDa and 100 kDa can be used.

The sorbent material according to the present invention is suitable for single-use bioreactors as well as reusable bioreactors. Specifically, single-use bioreactors can already be provided with a coating of the sorbent material, a patch or a semi-permeable pouch physically attached to the inside of the bioreactor upon manufacturing of the bioreactor to provide a user-friendly experience and an increased user efficiency. Further, the semi-permeable pouch, the tablet, the membrane and/or the filter cartridge comprising the sorbent material can be added by the user to a single-use or reusable bioreactor when setting up the cell culture.

In any case, once the cell culture is started, the sorbent material functions without any further external interaction with the cell culture, such as an exchange of medium. In this way, sterility problems caused by opening the bioreactor or exchanging medium can be avoided. High lactate transfer rates from the cell culture to the containments comprising the sorbent material are ensured by mixing (e. g. agitating, shaking, stirring or rocking the cell culture in a wave-like motion).

In a preferred embodiment of the present invention, the functional groups in the active material comprise boronate esters. These ester groups may be formed by condensation of a boronic acid with auxiliary compounds, such as diols or amino acids, preferably compounds that are beneficial for the health and/or growth of the cell culture. For example, these auxiliary compounds may be selected from energy substrates and other nutrients, such as carbohydrates, amino acids, polysaccharides, vitamins, nucleotides, or conditioning agents. Specific examples include α-D-glucopyranoside, α/β-D-glucofuranoside and L-glutamine.

In view of the above, according to the present invention, for example amino acids that can serve as nutrients or conditioners to cells of interest can be used. According to a preferred embodiment, they are "bidentate", i.e. they have two OH, two NH groups or one OH and one NH group.

Providing a sorbent material having such auxiliary compounds bound to the boronic acid moieties allow for a concerted release of the auxiliary compounds upon adsorption of the lactate. Accordingly, the sorbent material according to this embodiment has a two-fold beneficial impact on the cell culture. On the one hand, lactate, which has a cell toxic effect in higher concentrations, is removed from the cell medium. On the other hand, at the same time as adsorbing the lactate, the sorbent material releases the auxiliary compounds bound thereto, which are then available to the cells. As a result, the cell culture can be kept healthy for a longer period of time compared to the same cell culture without the sorbent material.

Conventionally, to extend the culture time, medium exchange is necessary. This can either be achieved by employing a semi-batch or batch reactor, where medium can be exchanged, or by using a perfusion reactor, where fresh medium is continuously fed, and metabolites are removed. However, these types of cell culture require sophisticated set-ups and carry the risk of introducing contaminants during the medium exchange.

The sorbent material according to the present invention is therefore particularly suitable for use in single-batch bioreactors, which are normally filled with the cell culture and left for a certain reaction time without opening or otherwise interfering with the reactor and its content.

The active material can comprise only one kind of boronate ester group, or it can comprise two or more different kinds of boronate ester groups each comprising a different auxiliary compound. In this manner, the cell culture can be provided with more than one kind of auxiliary compounds, such as, for example, an energy source, amino acids, or vitamins.

In this embodiment, in case the sorbent material is provided in the form of a semi-permeable pouch, bag or sachet, the membrane forming the pouch is selected so as to be permeable to the molecules released by the active material in addition to being permeable to lactate.

The working principle of the sorbent material according to this embodiment is illustrated below:

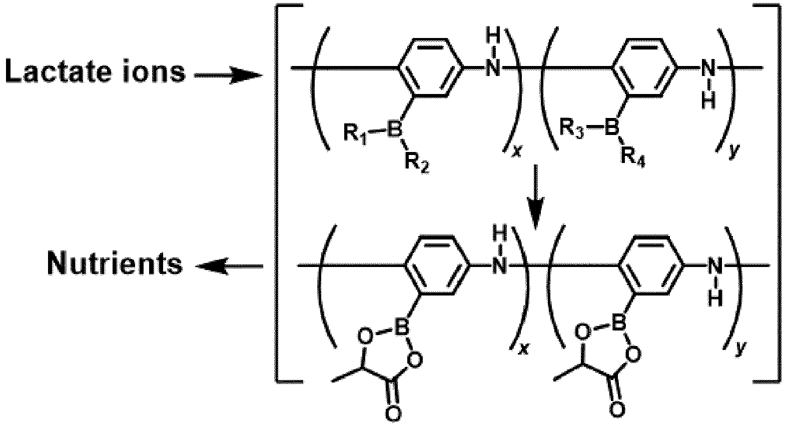

In this illustration, the groups R₁ and R₂ and/or R₃ and R₄, each taken together, represent an auxiliary compound that is bound to the respective boron atom. The indices x and y represent the fraction of the respective monomer units relative to the whole polymer where x + y equals 1 in the starting polymer. Upon exposure to lactate ions, the auxiliary compounds R₁/R₂ and/or R₃/R₄ are released from the active material into the environment and lactate is adsorbed to the active material.

The active material can be prepared in different ways. As a first example, the active material can be prepared by subjecting a polymer backbone carrying boronic acid groups or boronic acid ester groups to membrane dialysis in acidic or alkaline solutions in order to cleave existing ester bonds. In a next step, the so prepared compound is exposed to a solution of auxiliary compounds beneficial for the cell culture at higher pH. In case more than one auxiliary compound is used, the ratios of the different ester groups on the polymer backbone can be adjusted by solution concentrations and the thermodynamics of boronic ester exchange.

As a second example of the preparation of the active material, (co)polymerization of monomer units carrying boronic acid ester groups with auxiliary compounds beneficial for the cell culture can be performed. The monomers can be randomly polymerized with a statistical distribution of the different monomer types over the polymer chain. Alternatively, the copolymer can comprise distinct polymer blocks which are each made of different monomer units. In this case, the ratios of the different ester groups on the resulting polymer backbone can be adjusted according to the ratio of monomer units before polymerization.

The present invention further provides a method of cultivating cells in a bioreactor comprising a step of providing a sorbent material to the cell medium, the sorbent material being the sorbent material described above.

As explained before, the sorbent material may be added to the bioreactor before the cell culture is started or it can be added during the cultivation once or in regular intervals. However, for reasons of efficiency and avoiding contamination, it is preferred that the sorbent material is added before the cell culture is started and remains in the cell culture until the cell-culture process is stopped.

The bioreactor may be a single-use or reusable bioreactor and it may be operated as a single batch or semi-batch reactor.

The present invention further provides a method of using the above-described sorbent material in a bioreactor.

In summary, the present invention provides a lactate-adsorbing sorbent material. Lactate is continuously formed in cell cultures as a metabolite, which has an increasingly disruptive effect on the health of the cell culture. By providing the sorbent material to a cell culture, lactate coming into contact with the sorbent material can be bound, thereby reducing the concentration of lactate in the cell culture. If the functional groups on the polymeric backbone are previously loaded with a similarly small molecule required by the culture as a nutrient, then this can be used by the culture. Thus, a double positive effect is achieved, an interfering metabolite (lactate) is captured and a desired nutrient (e.g. a suitable carbohydrate) is supplied to the culture and this without the need to open the bioreactor containing the culture, which would otherwise be associated with sterility or contamination problems. In this way, the sorbent material according to the present invention can extend the lifetime, viability and target-protein productivity of the cell culture.

The present invention will be further illustrated in the following examples without being limited thereto.

### Examples

### Synthesis details for Example 1:

Poly(aminophenyl)boronic acid (PAPB) hemisulfate ester was prepared from the corresponding polylactate ester (PAPB-Lac), which in turn was synthesized from 3-aminophenylboronic acid and sodium lactate by adopting known procedures (N. V. Zaryanov, V. N. Nikitina, E. V. Karpova, E. E. Karyakina and A. A. Karyakin, Anal. Chem., 2017, 89, 11198-11202. A. G. MacDiarmid and A. J. Epstein, Faraday Discuss. Chem. Soc., 1989, 88, 317-332.).

In particular, 3-ammoniumphenylboronic acid hemisulfate (465 mg, 2.5 mmol) and sodium lactate (1.68 g, 15 mmol) were added to a 50-mL round-bottomed flask and charged with an aqueous 0.3 M H₂SO₄ solution (10 mL, 3 mmol). Ammonium persulfate (7.61 g, 33.3 mmol) was dissolved separately in 0.3 M H₂SO₄ (15 mL, 4.5 mmol) then added to the reaction flask at room temperature. The reaction mixture was stirred overnight to form a greenish-black suspension; the black solid was filtered through a Kirayama funnel (40Φ) and washed with deionized water (3 x 20 mL) then transferred onto filter paper and dried in air then vacuum. The final mass of dried PAPB-Lac ranged from 215 to 400 mg (up to 70% yield).

PAPB-Lac (200 mg) was suspended in deionized water (12 mL) inside a pouch made of membrane dialysis tubing comprised of benzoylated cellulose (MWCO 2000 Da). The pouch was dialyzed against deionized water (250 mL; 2 × 1 h), 0.3 M H₂SO₄ (250 mL; 2 × 1.5 h, 1 × 12 h), and deionized water (250 mL; 2 × 1 h), then collected by centrifugation/filtration and dried in air then vacuum under P₂O₅ to obtain 152 mg of dried PAPB hemisulfate ester. In this context, PAPB does not require drying and can be used immediately after dialysis.

## Claims

1. A lactate-adsorbing sorbent material for application in a bioreactor comprising an active material,
the active material having a polymeric backbone comprising one or more kinds of functional groups selected from boronic acid groups and boronic acid ester groups, and
wherein the sorbent material is in the form of a semi-permeable pouch, bag or sachet containing the active material, in the form of a reactor wall coating, in the form of a patch fixed to the reactor wall, in the form of a tablet, in the form of porous or non-porous beads or in the form of a membrane or filter cartridge.

2. The sorbent material according to claim 1, wherein the boronic acid ester groups stem from the reaction of a boronic acid group with a diol compound selected from carbohydrates, amino acids, vitamins, nucleotides or conditioning agents.

3. The sorbent material according to claim 1, wherein at least 75% of the repeating units of the polymer backbone carry a functional group.

4. The sorbent material according to claim 1, wherein the polymeric backbone is an aromatic polymer.

5. The sorbent material according to claim 1, wherein the aromatic polymer is polyaniline.

6. A method of cultivating cells in a bioreactor comprising a step of providing a sorbent material to the cell medium, the sorbent material comprising an active material,
the active material having a polymeric backbone comprising one or more kinds of functional groups selected from boronic acid groups and boronic acid ester groups, and
wherein the sorbent material is in the form of a semi-permeable pouch, bag or sachet containing the active material, in the form of a reactor wall coating, in the form of a patch fixed to the reactor wall, in the form of a tablet, in the form of porous or non-porous beads or in the form of a membrane or filter cartridge.

7. The method according to claim 6, wherein the boronic acid ester groups stem from the reaction of a boronic acid group with a diol compound selected from carbohydrates, amino acids, vitamins, nucleotides or conditioning agents.

8. The method according to claim 6, wherein at least 75% of the repeating units of the polymer backbone carry a functional group.

9. The method according to claim 6, wherein the polymeric backbone is an aromatic polymer.

10. The method according to claim 6, wherein the aromatic polymer is polyaniline.

11. A method of using a sorbent material in a bioreactor, the sorbent material comprising an active material,
the active material having a polymeric backbone comprising one or more kinds of functional groups selected from boronic acid groups and boronic acid ester groups, and
wherein the sorbent material is in the form of a semi-permeable pouch, bag or sachet containing the active material, in the form of a reactor wall coating, in the form of a patch fixed to the reactor wall, in the form of a tablet, in the form of porous or non-porous beads or in the form of a membrane or filter cartridge.
